# EUROPEAN PATENT SPECIFICATION

(11) **EP 0 521 562 B1**
(45) Date of publication and mention of the grant of the patent: **05.03.2003**
(21) Application number: 92201917.9
(22) Date of filing: 26.06.1992
(51) Int. Cl.: A61K 9/127

(54) **Vesicles in non-polar media**
Vesiculae in nichtpolaren Medien
Vésicules dans des milieux non-polaires

(30) Priority: 26.06.1991 EP 91201651
(43) Date of publication of application: 07.01.1993
(73) Proprietor: YAMANOUCHI EUROPE B.V., 2350 AC Leiderdorp (NL)
(72) Inventor: De Vringer, Tom, NL-2713 RX Zoetermeer (NL); Mollee, Hinderikus Marius, NL-2253 JL Voorschoten (NL)

(56) References cited:
- WO-A-86/02264
- WO-A-87/02582
- WO-A-90/06747
- US-A- 3 384 544
- JOURNAL OF THE AMERICAN CHEMICAL SOCIETY vol. 113, no. 3, 30 January 1991, WASHINGTON,DC, (US) pages 1051 - 1052 H. KUNIEDA ET AL. 'formation of reversed vesicles'
- PATENT ABSTRACTS OF JAPAN vol. 11, no. 43 (C-402)(2490) 7 February 1987

## Description

The present invention is concerned with novel vesicles, their preparation and their applications.

### BACKGROUND OF THE INVENTION

Several types of surfactant-based vesicles are known. Examples are liposomes, NIOSOMES® and "paucilamellar lipid vesicles". In fact, NIOSOMES® and "paucilamellar lipid vesicles" are special types of liposomes.

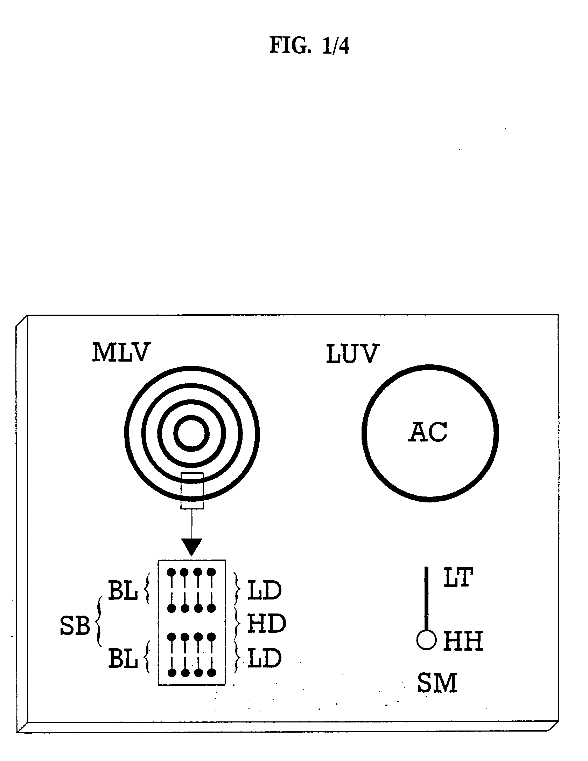

Liposomes are the most familiar vesicles and were described for the first time by Bangham in 1964 [A.D. Bangham, R.W. Horne, J. Mol. Biol. 8, 660 (1964)]. Liposomes consist of an aqueous core (AC, Figure 1) encapsulated by one or more spherically closed lamellar sheets. In turn, these lamellar sheets consist of bilayers (BL) of surfactant molecules (SM). Like the core of a liposome the external phase is water or an aqueous solution.

The lipophilic tails (LT) of the surfactant molecules in a bilayer are arranged in such a way that they are in close contact with each other and that contact between these lipophilic tails and the aqueous internal and external phases is avoided. On the other hand, the hydrophilic heads (HH) of the surfactant molecules are fully hydrated. In this way the system reaches the thermodynamically most favourable situation. If a liposome consists of more than one bilayer, the sequential bilayers (SB) are separated by aqueous layers. In case of Multilamellar Lipid Vesicles (MLV's) hydrophilic domains (HD) alternate lipophilic domains (LD).

Several types of liposomes can be distinguished. The classification is based on the size of the liposomes and the number of bilayers building up the vesicle. A Small Unilamellar Vesicle (SUV) is relatively small (e.g. 50 nm) and consists of only one bilayer enclosing an aqueous core. Large Unilamellar Vesicles (LUV's) may have sizes up to several micrometers. The critical size which determines whether a vesicle is called small or large is not well defined in the prior art.

Vesicles consisting of more than one bilayer are called Multilamellar Lipid Vesicles (MLV's). Sometimes the name "paucilamellar lipid vesicles" is used for MLV's which are built up of only a few (2-8) bilayers [see: WO 88/06883]. More details about the classification can be found in: N. Weiner et al., Drug Dev. Ind. Pharm., 15 (10) 1523-1554 (1989).

The surfactants building up the bilayers of a liposome can be ionic (e.g. phospholipids) and/or non-ionic (e.g. polyoxyethylene alkyl ethers). At first, the name NIOSOMES® was reserved for non-ionic surfactant vesicles, not containing any ionic surfactant molecules [R.M. Handjani-Vila et al. Int. J. Cosm. Sci., 1, 303-314 (1979)]. Nowadays, also ionic surfactant molecules may be incorporated in the bilayers of the NIOSOMES®, in order to improve stability.

Because of their aqueous cores, liposomes can be used as carriers for water-soluble substances. A solute which is present in the aqueous core is entrapped in the liposome for a certain time. The release from the vesicle can be passive by diffusion or leakage, or can be activated by trigger mechanisms. A possible trigger mechanism is for instance an increase in temperature resulting in a phase transition of the lipophilic chains of the surfactant molecules in the bilayers.

The retention of the solute in liposomes can, for example, advantageously be used in order to reduce the "first pass effect" of drugs in the liver, to reduce toxicity of substances, to enhance penetration of a substance into the skin, to achieve a sustained release of the solute, for drug targeting to a specific site, or to create a local environment in which the solute is shielded from a second substance in the external phase or in which the solute (e.g. an enzyme) can react with another substance (e.g. a substrate).

Several methods can be used to prepare the different types of liposomes. For a detailed review of the methods of preparation see: N. Weiner et al., Drug Dev. Ind. Pharm., 15 (10) 1523-1554 (1989).

One method is the "film-method". With this method the bilayer forming components are dissolved in an organic liquid (e.g. chloroform, diethyl ether, methanol) or a mixture of such liquids. This solution is rotated in a round-bottom flask, placed in a thermostated water bath, at reduced pressure. The organic solvents evaporate and a film of surfactant molecules is formed on the glass wall at the inside of the flask. The dry film is hydrated by adding an excess of water while shaking. In this way liposomes are formed. The aqueous dispersion of liposomes may be sonicated to achieve a smaller size and smaller size distribution.

Another method is the "reversed phase evaporation method". With this method the bilayer forming components are dissolved in an apolar organic liquid (e.g. chloroform, diethyl ether, isopropyl ether) or a mixture of such liquids. This apolar organic solution is mixed with water by e.g. sonication, forming an emulsion of the water-in-oil type. In this emulsion the surfactant is located at the oil-water interface. Subsequently the excess of organic solvent is removed at reduced pressure and the ratio organic solvent/ water (which is typically 3 or even more at the start) decreases. At this stage the bilayers are formed resulting in gelling of the mixture. Further removal of the organic solvent results in a collapse of the gel and the formation of vesicles (typically LUV's). The obtained product is an aqueous dispersion of vesicles with aqueous cores. After the method of preparation, these vesicles are named REV's (Reversed phase Evaporation Vesicles).

Several other methods can be used to prepare small batches of lipid vesicles. However, all known methods of preparation are related to one or more problems when it comes to upscaling of the process. These are problems of different kind, e.g. the removal of large quantities of explosive and toxic organic solvents, or the impossibility to sonicate large batches.

Irrespective of the method used, all stable lipid vesicles known to date are formed upon hydration of the surfactant, by adding an excess of water or an aqueous solution to a lipid phase containing the surfactant and optionally other lipid soluble substances (e.g. cholesterol). The resulting vesicles, whether it are liposomes, NIOSOMES®, SUV's, LUV's, REV's, MLV's or "paucilamellar lipid vesicles", all are dispersed in an aqueous, polar phase during the manufacturing process.

On preparing the liposomes only a part of the aqueous solution, which may contain for example a water soluble drug, is encapsulated. Therefore, the water-soluble substances are distributed over the aqueous internal and external phases.

Often cholesterol is incorporated into the bilayers to modify the properties thereof. In some compositions cholesterol is even an essential component for the formation of the liposomes.

Theoretically, also other lipophilic compounds, for instance drugs, can be incorporated into the lipophilic domains of the bilayers. Of course, more lipophilic substance can be incorporated into MLV's than into SUV's or LUV's. However, the bilayers have a highly ordered structure. Very few substances show a good structural fit with these bilayers (cholesterol does). Therefore, in practice very few substances can be incorporated to significant amounts into the lipophilic domains of the bilayers.

Moreover, the total volume of the lipophilic domains of the bilayers is small, because of the limited amount of surfactant (typically 30 µmol per gram of dispersion) that can be used at the preparation of the vesicles. An unusual high quantity of surfactant, viz. 200 µmol per gram, was used by H. Talsma et al. [Drug Dev. Ind. Pharm., 15 (2) 197-207 (1989)].

In turn, the limitation of the amount of surfactant, strongly limits the amount of substance that can be incorporated into the lipophilic domains of the bilayers. Therefore, the vesicles known to date are usually not suitable to encapsulate a lipophilic compound. For more details about the encapsulation of substances see: L.D. Mayer et al., Chem. & Phys. Lipids, 40 (1986) 333-345.

Because of their aqueous interiors, liposomes are more suitable to encapsulate water-soluble compounds, especially SUV's and LUV's, the latter showing the best encapsulating efficiencies. Unfortunately, SUV's and LUV's show a much worse retention of the entrapped solute.

Due to the methods of preparation of the vesicles known to date, a substantial amount of aqueous phase is not encapsulated. As a result, also a substantial amount of water-soluble substance is not encapsulated, reducing the encapsulating efficiency to a large extent.

Several disadvantages are related to this low encapsulating efficiency. In the first place, for most applications, the not encapsulated solute has to be removed from the external phase. This means that at least one additional manipulation has to be carried out. For lab-scale quantities of liposome dispersions, this can be done by dialysis. For quantities on an industrial scale it is a major drawback.

In the second place a substantial amount of the compound is lost for its purpose and cannot function in the proper way, which it should do if it were encapsulated. Especially for expensive substances (e.g. peptides) this spill is an important disadvantage.

Because of the disadvantages related to a low encapsulating efficiency, a lot of research is being focused on this problem. Though improvements have been made, the encapsulating efficiency is still far from ideal. Furthermore, the additional techniques which have to be employed in order to improve the encapsulating efficiency (dehydration and rehydration of the liposomes, freeze-thaw procedures and for ionic solutes active entrapment methods) are not suitable for industrial purposes because of the high costs or upscaling problems.

H. Kunieda et al. [J. Am. Chem. Soc. 113(3) 1051-1052 (1991)] has described the formation of reversed vesicles, consisting essentially of the hydrophilic surfactant tetra-ethyleneglycol dodecylether in dodecane. The addition of about 2.5 water molecules/ ethyleneoxide-unit was found to be essential for the formation of the vesicles, which however were found to coalesce and revert back to a lamellar liquid crystalline phase over a period of hours to days.

### SUMMARY OF THE INVENTION

It has now been found that dispersions of stable vesicles in a non-polar phase can be made. Said vesicles, which encapsulate a non-polar phase, comprise a surfactant or a mixture of surfactants selected from the group consisting of sucrose fatty acid esters, and, if necessary, dependent on the choice of the surfactant(s) and non-polar phase, also a lipophilic stabilizing factor and occasionally a hydrophilic stabilizing factor too.

The invention provides methods for the preparation of said dispersions of vesicles, the methods comprises preparing a mixture of the surfactant(s), the lipophilic stabilizing factor, the non-polar phase and optionally the hydrophilic stabilizing factor, optionally at an elevated temperature and/or by the use of organic solvents, which are subsequently evaporated. Examples thereof are a mixing method, an evaporation method and a film method.

Said dispersions of vesicles can be advantageously incorporated into pharmaceutical, cosmetical, food and pesticide preparations and into preparations for treating surfaces in general.

### LEGENDS TO THE FIGURES

Figure 1 is a schematic representation of a Multilamellar Lipid Vesicle and of a Large Unilamellar Vesicle.
- MLV =: Multilamellar Lipid Vesicle
- LUV =: Large Unilamellar Vesicle
- AC =: Aqueous core of liposome
- BL =: Bilayer of surfactant molecules
- SB =: Sequential Bilayers separated by an aqueous layer
- LD =: Lipophilic domain of bilayer consisting of lipophilic tails of surfactant molecules
- HD =: Hydrophilic domain of bilayer consisting of aqueous layer plus hydrophilic heads of surfactant molecules
- SM =: Surfactant molecule
- LT =: Lipophilic tail of surfactant molecule
- HH =: Hydrophilic head of surfactant molecule

Figure 2 is a photograph of the vesicles in liquid paraffin of Example 1.1, obtained by the Freeze Fracture Electron Microscopy (FF-EM) technique.

Figure 3 is a graphical representation of the budesonide release from cream formulations 5.1 and 5.2 through a cellulose membrane as a function of the square root of time.

Figure 4 is a graphical representation of the blanching scores caused by the cream formulations 5.3, 5.4 and 5.5 as a function of time after washing off the preparations from the arms.

### DETAILED DESCRIPTION OF THE INVENTION

It has now been found that stable vesicles in a non-polar phase can be prepared. These vesicles, which encapsulate a non-polar phase, comprise one or more surfactants selected from the group consisting of sucrose fatty acid esters, and if necessary, dependent on the choice of the surfactant(s) and non-polar phase, a lipophilic stabilizing factor and occasionally a hydrophilic stabilizing factor too.

The stability of the vesicles according to the invention may be determined by microscopical comparison of the product, shortly after preparation and at any time thereafter, which is at least one week, but preferably several months or years. Stable means that no changes can be observed in the microscopical image and/or in the structural integrity of the vesicles. Stability in the course of time preferably is followed by comparing photographs of the microscopical image, taken at several points of time. The use of a QUANTIMET®-measuring device seems to be most appropriate in this respect.

Said vesicles, as a rule, have a size of between 20 and 100,000 nm, preferably between 40 and 25,000 nm, more preferably between 100 and 5,000 nm. They consist of a non-polar core encapsulated by 1-10,000, preferably 5-2500, more preferably 10-500 spherically closed lamellar sheets. In its turn, these lamellar sheets consist of bilayers of the surfactant molecule(s), the polar head groups of which forming the inner part of the bilayer and the non-polar chains of the same molecule(s) protruding in the non-polar phase.

The lipophilic stabilizing factor is a molecule characterized by a relatively massive elongated non-polar part and a small polar part. After incorporation of said molecule into the vesicles it will become located in the lipophilic domain of the bilayers, i.e. between the non-polar chains of the surfactant molecules, thereby modifying the hydrocarbon packing stress. It will be appreciated by a person skilled in the art that if the hydrocarbon packing stress is at an appropriate level, the incorporation of the lipophilic stabilizing factor is not required. It has been demonstrated that no such factor is needed e.g. for the preparation of a dispersion of stable vesicles consisting of sucrose-esters in non-polar vehicles such as liquid paraffin and volatile silicone oil. Nevertheless, if said factor would be present, stable vesicles will also be formed according to the present inventors' experience. Examples of the lipophilic stabilizing factor are:
- sterols, such as (partially) hydrogenated 10,13-dimethyl cyclopentaphenanthrenes, optionally substituted by e.g. hydroxyl and carboxylic acid, and esters thereof;
- retinoids, such as retinoic acid;
- branched fatty alcohols, such as 2-hexyl octyl ethanol;
- straight or branched chain (number of C-atoms: 4 or above) saturated aliphatic alcohol esters of aliphatic or aromatic dicarboxylic acids (number of C-atoms: 4 or above), such as dioctyl phthalate;
- saturated and unsaturated fatty acids, such as palmitic acid, stearic acid and linoleic acid; and
- straight and branched fatty acid esters with polyhydroxylic compounds, such as propyleneglycol dipelargonate and diglycerol diisostearo stearate,
but preferably cholesterol is used.

The lipophilic stabilizing factor can be used in a concentration of up to 200 wt%, preferably up to 100 wt% and most preferably 10-50 wt%, based on the amount of surfactant(s) used.

It will be appreciated by a person skilled in the art that the polar part(s) of the surfactant(s) building the structure of the vesicle may be able to interact in the hydrophilic domains of the bilayers in such a way as to sufficiently stabilize the vesicles. This is the case when using sucrose-esters. However, dependent on the choice of the surfactant, the presence of a hydrophilic stabilizing factor in the vesicles may be required for obtaining stable vesicles.

The hydrophilic stabilizing factor is a small polar molecule (having a molecular weight of up to 150), which has the ability of forming hydrogen-bridges. After incorporation into the vesicles such molecules will be located in the hydrophilic domains of the bilayers, as to contribute to the stability of the vesicles. Said molecule may be water or a compound having one or more functional groups able to form hydrogen-bridges such as a hydroxyl, a (mono- or di-substituted) amino and a carboxylic acid group. Examples of the last-mentioned compound are ethanol and ethanolamine.

The hydrophilic stabilizing factor can be used in a concentration of up to 50 wt%, preferably up to 35 wt% and most preferably 25 wt%, based on the amount of surfactant(s) used.

The vesicles according to the present invention as compared with vesicles in an aqueous phase have a very high encapsulating capacity (which is the amount of encapsulated active ingredient per gram of dispersion) for hydrophilic as well as for lipophilic compounds. The manufacturing process allows a very high encapsulating efficiency (which is the amount of encapsulated active ingredient relative to the total amount of active ingredient in the dispersion) in the vesicles according to the present invention for hydrophilic substances, as compared to the encapsulating efficiency in vesicles in an aqueous phase.

The advantages of the entrapment of hydrophilic as well as lipophilic substances into the vesicles according to the present invention, are broadly speaking the same as those observed after the entrapment of solutes into e.g. liposomes:
- reduction of the first pass effect of drugs in the liver;
- reduction of the toxicity of substances;
- enhancement of the penetration of a substance into the skin;
- achievement of a sustained release of the solute;
- enhancement of the amount of drug released at a specific site;
- creation of a barrier to physically separate 2 incompatible substances, that have to be incorporated into one dosage-form.

Due to the manufacturing method and the choice of the components the vesicles of the present invention also offer some additional advantages in product development, over those already offered by the different types of liposomes, as outlined above:
- the concentration of surfactant, that can be used, is much higher (e.g. up to 1400 µmol per gram of dispersion) than that which can be used during the preparation of the several types of liposomes;
- accordingly, the amount of encapsulated substance (hydrophilic and lipophilic) can be much higher;
- the vesicles are very suitable for the incorporation of substances which are not stable in the presence of water or oxygen, under the influence of light or under certain pH-conditions of the polar phase, because the vesicles of the invention can be made without using any water; and
- when no water is used, the vesicles can be prepared with easily hydrolysible ester surfactants, which cannot be used for the preparation of stable liposomes. After administration of these ester surfactants they will hydrolyse upon contact with water. Another advantage of said biodegradable esters is that they are less toxic.

The surfactants according to the invention are:
sucrose fatty acid esters and mixtures thereof (e.g. WASAG ESTER 7® and WASAG ESTER 15®)

The concentration of surfactant(s) will vary according to the surfactant(s) and non-polar excipient(s) used and the application aimed at.

The concentration of total surfactant by weight, based on the weight of the vesicles-containing dispersion, is, as a rule, between 0.5 and 70%, preferably between 5 and 40%, more preferably between 10 and 30%.

The non-polar phase can be formed from non-polar excipients, which are either solid or liquid at room temperature. Also, mixtures of such non-polar excipients can be advantageously used.

A non-polar excipient according to the present invention is not miscible with water in all proportions at a temperature between 0°C and 100°C, at atmospheric or reduced pressure, or having a value for the dielectric constant (ε) less than 20.0, at 25°C and atmospheric pressure.

In case the non-polar phase has been formed from a mixture of non-polar excipients, said mixture should not be miscible with water in all proportions at a temperature between 0°C and 100°C, at atmospheric or reduced pressure, or at least one of the components of the mixture has a value for the dielectric constant (ε) less than 20.0, at 25°C and atmospheric pressure.

Suitable non-polar excipients are for example:
- straight chain aliphatic alcohols, the number of carbon atoms being 3 or above;
- waxes, such as jojoba-oil and carnauba wax;
- hydrocarbons, such as liquid paraffin, white soft paraffin, hard paraffins, squalene and isoparaffins;
- ketones, such as 5-methylhexaan-2-on;
- synthetic esters, such as cetylpalmitate;
- glycerol tri-esters of higher saturated and unsaturated fatty acids having 10-30 carbon atoms, such as glyceryltrilaurate and hydrogenated castor oil;
- vegetable oils such as coconut-oil and peanut-oil;
- silicone oils, such as the volatile silicone oils (e.g. octamethyl cyclotetrasiloxane (ABIL K 4®), decamethyl cyclopentasiloxane, dodecamethyl cyclohexasiloxane).

Preferably, non-polar excipients from the group of the hydrocarbons and from the group of the volatile silicone oils are used. More preferably mineral oil (=liquid paraffin) and octamethyl cyclotetrasiloxane are used.

The choice of the surfactant(s) as well as of the non-polar excipient(s) depends not only on the substances per se, but also is dependent on the intended application, and of the preferred manufacturing process.

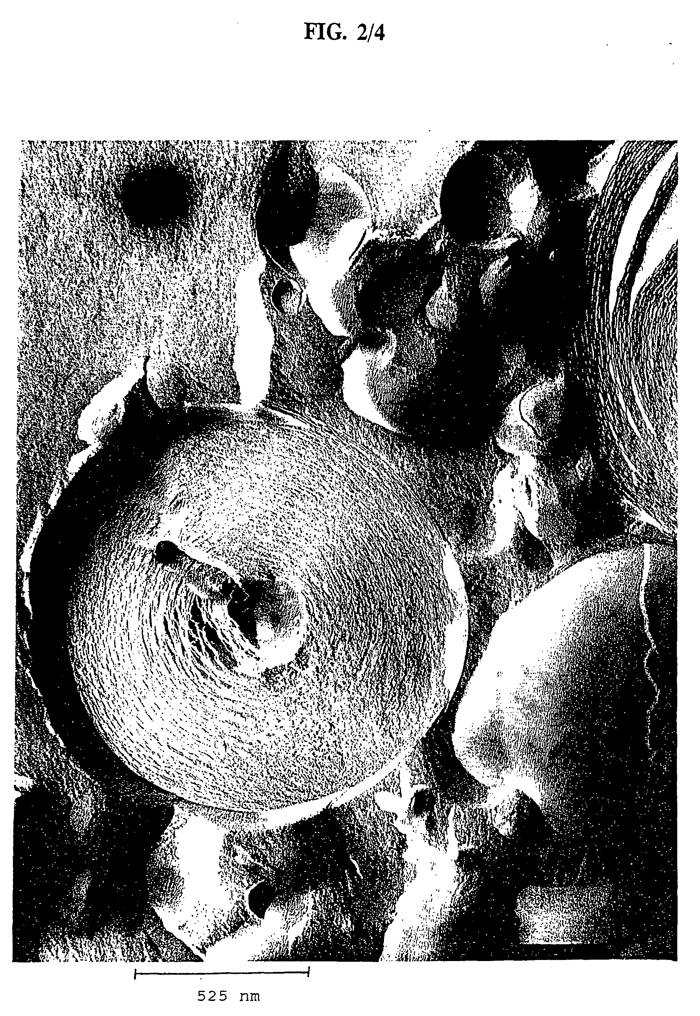

The vesicles according to the invention can be characterized by methods commonly known in the art. Examples of these methods are: Freeze Fracture Electron Microscopy (FF-EM), Light Scattering techniques, such as Dynamic Light Scattering (DLS) especially for small vesicles, and X-ray diffraction and neutron diffraction techniques. The vesicles, described in the examples hereafter, were characterized by FF-EM and by means of polarized light microscopy. This last-mentioned technique has been very useful in detecting crystals of the active substances, used in the examples. An example of a photograph, obtained by the FF-EM technique, is shown in Figure 2.

The dispersion of vesicles according to the present invention can be prepared by methods, which comprise preparing a homogeneous mixture of the surfactant(s), the lipophilic stabilizing factor, the non-polar phase and optionally the hydrophilic stabilizing factor, optionally at an elevated temperature and/or by the use of organic solvents, which are subsequently evaporated. Examples thereof are a mixing method, an evaporation method and a film method, which are discussed hereafter. The two last-mentioned methods are allied to methods for the preparation of different types of vesicles in aqueous vehicles as known in the art: the reversed phase evaporation method and the film method respectively.

The mixing method comprises the steps of:
- mixing, without high shear being required, all components (which are the surfactant(s) and non-polar excipient(s) and may also be the lipophilic stabilizing factor and the hydrophilic stabilizing factor) at a temperature at or above that at which all ingredients are molten or dissolved; and
- if necessary cooling to room temperature, while stirring.

Cooling can be carried out actively with methods known in the art. Active substances, such as drugs and colouring agents, can be incorporated into the vesicles, by adding them to the non-polar phase before mixing and heating, if necessary.

The evaporation method comprises the steps of:
- dissolving the surfactant(s), the lipophilic stabilizing factor and, if present, the hydrophilic stabilizing factor in a (mixture of) polar organic solvent(s), if necessary at an elevated temperature;
- adding the clear solution of surfactant(s) to the non-polar phase at a temperature at or above the temperature of the surfactant solution;
- preferably agitating the resulting mass and allowing the polar organic solvent(s) to evaporate, if necessary under reduced pressure; and
- if necessary cooling to room temperature, preferably whilst stirring.

When using the evaporation method, the choice of a (mixture of) polar solvent(s) is essential. It is important to choose such a (mixture of) solvent(s) as to enable the dissolution of the surfactant(s) and the stabilizing factor(s) at a suitable temperature. A further requirement which has to be met is the non-mixability of said (mixture of) solvent(s) with the non-polar phase. Preferred polar solvents are ethanol and acetone.

Having obtained a clear solution of the surfactant(s) and the stabilizing factor(s), this is added to the non-polar phase. To prevent a delay in the manufacturing process the temperature of the non-polar phase preferably is the same as that of the solution. During the subsequent mixing of the two phases the polar phase is allowed to evaporate. This process can be accelerated by reducing the pressure.

When the polar phase is completely evaporated the non-polar phase is cooled to ambient temperature, if necessary. This cooling can be carried out actively with methods known in the art.

Preferably active substances, such as drugs, which are to be incorporated into the vesicles, will be dissolved in the polar phase, unless the solubility in the non-polar phase would be much better. The same holds for the lipophilic stabilizing factor and the hydrophilic stabilizing factor.

The film method comprises the steps of:
- dissolving the surfactant(s), the lipophilic stabilizing factor and, if present, the hydrophilic stabilizing factor in a (mixture of) organic solvent(s);
- removing the organic solvent, using the rotational evaporation technique;
- solvatating the residue in the non-polar phase, if necessary at a temperature above the melting point of the surfactant(s); and
- if necessary cooling to room temperature.

When the film method is used the (mixture of) surfactant(s) and the stabilizing factor(s) are dissolved in a suitable (mixture of) organic solvent(s), easy to remove by evaporation. Preferred organic solvents are dichloromethane, chloroform and methanol. The solution, comprising the surfactant(s) and the stabilizing factor(s), is transferred to a roundbottom flask. After the complete evaporation of the solvent(s) by a rotational evaporation technique, the film which has been formed at the inside of the flask, is solvatated with a non-polar phase at a temperature above the melting point of the surfactant(s). After finishing the solvatating process, the dispersion of vesicles in the non-polar medium is cooled to ambient temperature, if necessary. This cooling can be carried out actively with methods as known in the art.

Preferably the active substances, such as drugs and colouring agents, which are to be incorporated into the vesicles, are added to the solution, comprising the surfactant(s), unless the solubility of those substances in the non-polar phase would be much better than in the solution of surfactant(s). The same holds for the lipophilic stabilizing factor and the hydrophilic stabilizing factor.

It will be appreciated that the choice of the method is determined by the components of the vesicles, the batch size to be achieved and the physico-chemical properties of the surfactant(s), the (biologically) active compound(s), the additives and the non-polar excipient(s) such as melting point, solubility in different media, volatility, behaviour at elevated temperatures and so on.

After having obtained a dispersion of vesicles according to one of the above-mentioned method, additional production steps comprise:
- reducing the size of the vesicles by methods known in the art (sonication, extrusion, microfluidization);
- polymerizing the vesicles in case a polymerizable surfactant is used by methods known in the art;
- removing the non-polar excipient(s) to obtain an instant preparation
- adding to the vesicles in non-polar media an appropriate aqueous or non-aqueous polar liquid, a gel based on such liquids, a liquid or semisolid lipid, or a mixture of any of the above, to make a solution, a gel, a water-in-oil emulsion, an oil-in-water emulsion, or an ointment;
- incorporating hydrophilic or lipophilic substances, such as drugs and colouring agents, into the vehicles, which comprise a dispersion of vesicles according to the invention.

Aqueous or non-aqueous polar liquids can be added to the dispersions of vesicles according to the invention, without disturbing the structure of the vesicles. However, due to the polar character of the vehicle to be added, the outer layer of the vesicles could get lost. Accordingly, for this kind of preparations it is a prerequisite to use dispersions containing multilamellar vesicles.

Liquid or semi-solid lipids may be mixed, as such or in a w/o or o/w emulsion, with the vesicles according to the invention. When a w/o or an o/w emulsion is used, to be mixed with the vesicles according to the invention, it will also contain an appropriate w/o or o/w emulsifier, as known in the art of making such w/o or o/w emulsions. Said emulsifiers have to be chosen carefully as to avoid the destruction of the vesicles.

The dispersions of vesicles of the present invention and/or the vehicles comprising said dispersions may also contain buffers, preservatives, anti-oxidants, moisturizers, penetration enhancers, UV absorbers, dyes, fragrances, flavours, sweeteners, pesticides, insect repellants etc..

As already mentioned, drugs may be advantageously incorporated into the vesicles and/or into the vehicles comprising the dispersion of vesicles according to the invention. As there seems to be no limitation with respect to the route of administration for the preparations mentioned, a variety of drugs can be incorporated therein, such as e.g. steroidal compounds, dithranol, nicotinic acid and derivatives thereof, retinoids, peptides, anti-inflammatory agents, anti-proliferative agents, antimicrobials and vitamins. This list of drugs should not be considered as a limitative enumeration. For practical reasons there is a preference for low-dose active ingredients to be incorporated into the vesicles. The vesicles in non-polar phase according to the present invention seem especially suited for the incorporation of drugs, which structurally resemble one of the components of the vesicles such as the steroidal compounds and vitamin A acid or derivatives.

The preference for the organic solvents to be used is determined for a great deal by the application aimed at: in certain categories of products, especially in the pharmaceutical, cosmetical, food and pesticide processing field, no significant amounts of certain organic solvents in the final products are allowed.

Especially if the dispersions of vesicles according to the present invention are used for the preparation of pharmaceutical dosage-forms, the components thereof should meet high standards regarding purity. The choice of these surfactant(s) and non-polar excipient(s) further depends on whether the pharmaceutical preparation, comprising the dispersion of vesicles according to the invention, is meant to be applied via the oral, the topical, the nasal, the rectal, the pulmonal or the parenteral (i.e. intravenous, intramuscular or subcutaneous) route.

However, the application of the dispersions of vesicles according to the present invention is not considered to be limited to pharmaceutical purposes. The incorporation of said dispersion of vesicles into for example cosmetic, food and coating preparations for the treatment of surfaces in general is thought to be of great value.

The applications of the vesicles according to the invention may be based on the following concepts:
- empty vesicles in a vehicle without any additional active ingredient;
- empty vesicles in a vehicle to which an active ingredient has been added;
- loaded vesicles in a vehicle without any additional active ingredient;
- and loaded vesicles in a vehicle to which an active ingredient has been added.

Examples for products, based on the vesicles according to the invention are:
- a cleansing product for the removal of grease and dirt;
- a detoxifying product for the removal of undesired toxic substances from the body;
- a product in which two incompatible compounds have been incorporated e.g. a cream, containing salicylic acid and vesicles, loaded with a corticosteroid;
- a product in which a fast and a sustained release of the active ingredient are combined;
- encapsulation of water-soluble moisturizers in cosmetic preparations;
- encapsulation of vitamin C in fruit juices;
- encapsulation of nicotine in chewing gum; and
- a product, comprising empty vesicles, built up from essential fatty acids, and a corticosteroid.

The following examples further illustrate the invention.

### EXAMPLES

### Example 1

### Production of dispersions of vesicles in a non-polar medium

**1.1.** Vesicles in liquid paraffin were made as follows:
   6 g of WASAG ESTER 15® and 600 mg of cholesterol were dissolved in 200 ml of acetone. To the resulting solution 300 g of liquid paraffin were added and this mixture was vigorously stirred at 70°C, using a TURRAX® homogenizer, in order to remove the solvent acetone by evaporation. The resulting dispersion was cooled to roomtemperature whilst stirring. The polarized light-microscope image of the resulting dispersion revealed the presence of a multiplicity of Maltese crosses, which may be considered as a measure for the amount of vesicles. The resulting (multilamellar) vesicles consisted of more then 20 bilayers (concluded from FF-EM photographs, see Figure 2) and had a mean diameter of 800 nm (calculated from these FF-EM photographs).
**1.2.** Another batch of vesicles in liquid paraffin was produced as follows:
   70 g of liquid paraffin was heated and degassed at 80°C. 30 g of WASAG ESTER 15® en 3 g of cholesterol were dissolved in 80 g of ethanol at 70°C. The clear ethanolic solution was mixed with the liquid paraffin using a stirrer at 150 rpm. The ethanol was removed under reduced pressure at 80°C. It was assumed that all ethanol was removed when the foaming of the dispersion disappeared. The resulting dispersion of vesicles was cooled under reduced pressure to 22°C. The resulting (multilamellar) vesicles consisted of more then 20 bilayers (concluded from FF-EM photographs) and had a mean diameter of 800 nm (calculated from these FF-EM photographs).
   The vesicles were stable for at least 1 year.
**1.3.** Another batch of vesicles was produced in the same way -and using the same ingredients- as 1.2., with the following amounts:
   50 g of WASAG ESTER 15® and 5 g of cholesterol dissolved in ethanol at 70°C and 250 g of liquid paraffin.
**1.4.** Another batch of vesicles was made in a volatile silicon oil as follows:
   70 g of ABIL K4® silicon oil, 30 g of WASAG ESTER 15® and 3 g of cholesterol were mixed at 80°C using a stirrer at 150 rpm. After 45 minutes the mixture was cooled to 22°C. The resulting vesicles had a mean diameter of 1 µm.
**1.5** Other lipid vesicles were made in a volatile silicon oil as follows:
   30 g of ABIL K4® silicon oil, 3 g of WASAG ESTER 15® and 0.3 g of palmitic acid were mixed at 85°C using a stirrer at 300 rpm. After 10 minutes the mixture was cooled to ambient temperature. The resulting lipid vesicles had a mean diameter of 1 µm. If palmitic acid was not added, the sample showed an large amount of aggregated lipid vesicles and non-vesicular material.
**1.6** Other lipid vesicles were made in a volatile silicon oil as follows:
   20 g of ABIL K4® silicon oil, 1 g of WASAG ESTER 15® and 0.1 g of retinoic acid were mixed at 85°C using a stirrer at 300 rpm. After 10 minutes the mixture was cooled to ambient temperature. The resulting lipid vesicles had a mean diameter of 1 µm. If retinoic acid was not added, the sample showed an large amount of aggregated lipid vesicles and non-vesicular material.
**1.7** Other lipid vesicles were made in a volatile silicon oil as follows:
   20 g of ABIL K4® silicon oil, 1 g of WASAG ESTER 15® and 0.1 g of propyleneglycol-dipelargonate were mixed at 85°C using a stirrer at 300 rpm. After 10 minutes the mixture was cooled to ambient temperature during sonification. The resulting lipid vesicles had a mean diameter of 1 µm. If instead of propyleneglycol-dipelargonate 0.1 g of diglyceryl-di-isostearo-stearate was used, the same amount of vesicles was obtained. If WASAG ESTER 15® alone was used, the sample showed a large amount of aggregated lipid vesicles and non-vesicular material.
**1.8** Other lipid vesicles were made in a volatile silicon oil as follows:
   10 g of ABIL K4® silicon oil, 1 g of WASAG ESTER 15® and 0.33 g of 2-hexyl-octyl-ethanol were mixed at 90°C using a stirrer at 300 rpm. After 10 minutes the mixture was cooled to ambient temperature during sonification. The resulting lipid vesicles had a mean diameter of 1 µm. If instead of 2-hexyl-octyl-ethanol 0.33 g of dioctylphtalate was used, the same amount of vesicles was obtained. If WASAG ESTER 15® alone was used, the sample showed a large amount of aggregated lipid vesicles and non-vesicular material.

### Example 2

### Production of dispersions of loaded vesicles in a non-polar medium

**2.1.** Vesicles loaded with disodium-fluorescein were made in the same way as 1.1, using the following ingredients:
   65 mg of disodium-fluorescein, 20 g of WASAG ESTER 15® and 2 g of cholesterol dissolved in 80 g of ethanol at 70°C and 100 g of liquid paraffin.
   The resulting vesicles had a diameter of 1 µm and showed a bright fluorescence typical for disodium-fluorescein. No crystals of disodium-fluorescein have been seen in this dispersion (examined by a polarized light microscope). Because of the extremely low solubility of disodium-fluorescein in liquid paraffin it was concluded that all disodium-fluorescein was encapsulated within the vesicles.
**2.2.** Another batch of vesicles loaded with the corticosteroid budesonide was made in the same way as 1.1, using the following ingredients:
   381 mg of budesonide, 50 g of WASAG ESTER 15® and 5 g of cholesterol dissolved in 100 ml of ethanol at 70°C and 250 g of liquid paraffin at 80°C.
   The resulting vesicles had a diameter of 1 µm. No crystals of budesonide have been seen in the sample. Because of the extremely low solubility of budesonide in liquid paraffin it was concluded that all budesonide was encapsulated within the vesicles.
**2.3.** Yet another batch of vesicles loaded with gramicidine D was made in the same way as 1.1, using the following ingredients:
   100.6 mg of gramicidine D, 20 g of WASAG ESTER 15® and 2 g of cholesterol dissolved in 100 ml of ethanol at 70°C and 100 g of liquid paraffin at 80°C.
   The resulting vesicles had a diameter of 1 µm. No crystals of gramicidine D have been seen in the sample. Because of the extremely low solubility of gramicidine in liquid paraffin it was concluded that all gramicidine D was encapsulated within the vesicles.
**2.4.** Yet another batch of vesicles loaded with the corticosteroid hydrocortison-17-butyrate was made in the same way as 1.1, using the following ingredients:
   250 mg of hydrocortisone-17-butyrate, 30.1 g of WASAG ESTER 15® and 3 g of cholesterol dissolved in 50 ml ethanol at 70°C and 70 g of liquid paraffin at 80°C.
   The resulting vesicles had a diameter of 1 µm. No crystals of hydrocortisone-17-butyrate have been seen in the sample. Because of the extremely low solubility of hydrocortison-17-butyrate in liquid paraffin it was concluded that all hydrocortisone-17-butyrate was encapsulated within the vesicles.
**2.5.** Yet another batch of vesicles loaded with hydrocortisone-17-butyrate was made in the same way as 1.3 using the following ingredients:
   70 of ABIL K4®, 30 g of WASAG ESTER 15®, 3 g of cholesterol and 0.25 g of hydrocortison-17-butyrate.
   The resulting vesicles had a mean diameter of 1 µm. No crystals of hydrocortisone-17-butyrate have been seen in the sample. Because of the extremely low solubility of hydrocortisone-17-butyrate in ABIL K4® it was concluded that all hydrocortisone-17-butyrate was encapsulated within the vesicles.
**2.6.** Vesicles loaded with methyl-p-hydroxybenzoate (NIPAGIN M®) were made in the same way as 1.1., using the following ingredients:
   400 mg of NIPA M®, 20 g of WASAG ESTER 15® and 2 g of cholesterol dissolved in 80 g of ethanol at 70°C and 177.6 g of liquid paraffin.
   The resulting vesicles had a mean diameter of 1µm. There were no crystals of NIPA M® present in this sample (examined by a polarized light microscope).
   1 g of the sample was centrifuged at 3000 RPM for 10 minutes using a HERAEUS CHRIST MINIFUGE 2 centrifuge to separate the vesicles from the external paraffin phase.
   50 µl of the clear external phase was extracted with 2 ml of a 75% propyleneglycol/water mixture. The UV absorption of the extract at 274.8 nm was measured using a SHIMADZU UV-160 spectrophotometer. The amount of NIPA M was calculated using a calibration curve of 6 calibration solutions. The concentration of NIPA M found in the external phase was 46.8 µg/ml (N=2), the measured saturation concentration of NIPA M in liquid paraffin is 55 µg/ml.
   It was calculated that 97.3% (N=2) of the total amount of NIPA M in the sample was encapsulated within the vesicles.

### Example 3

### Production of a conventional cream, and of a cream containing a dispersion of empty vesicles

**3.1.** A conventional cream was produced as follows:
   3000 g of white soft paraffin, 1200 g of liquid paraffin, 1440 g of cetylstearyl alcohol and 40 g of methyl-p-hydroxybenzoate (NIPAGIN M®) were heated together at 70°C. 360 g of CETOMACROGOL 1000®, 84 g of anhydrous citric acid and 56 g of anhydrous tri-sodium-citrate were dissolved in 13820 g of water at 70°C. Both phases were mixed together, concurrently using a stirrer at 200 rpm and a TURRAX® homogenizer at 2000 rpm. The dispersion was cooled under reduced pressure to 22°C.
**3.2.** A cream containing vesicles was produced as follows:
   50 g of the dispersion according to 1.2 was mixed with 200 g of the cream according to 3.1 for 30 minutes at 20°C with a stirrer at 50 rpm. After 3 months the vesicles were still homogeneously distributed over the cream (observed by a polarized light microscope).

### Example 4

### Production of a conventional gel, and a gel containing a dispersion of empty vesicles

**4.1.** A conventional gel was produced by dispersing 10 g of CARBOPOL 934® in 990 g of water. The pH of the resulting suspension was adjusted to pH = 4.0 with triethanolamine.
**4.2.** A gel containing vesicles was produced by handmixing 0.8 g of the dispersion according to 1.2 with 3.2 g of the gel according to 4.1 in a mortar.
   The resulting gel had a creamy appearance. The vesicles were homogeneously distributed over the gel (observed by a polarized light microscope). The vesicles were stable for at least 1 year.

### Example 5

### Production of creams, containing a dispersion of vesicles and a medicament (free or encapsulated in the vesicles) or a medicament alone

**5.1.** A cream containing budesonide encapsulated in vesicles was produced by mixing (with a stirrer at 50 rpm) during half an hour 50 g of the dispersion according to 2.2 with 200 g of the cream according to 3.1.
**5.2.** A cream containing free budesonide and vesicles was produced by mixing (with a stirrer at 50 rpm) during 30 minutes 50 g of the cream according to 3.2 with 12.5 mg of budesonide.
**5.3.** A cream containing hydrocortison-17-butyrate encapsulated in vesicles was produced by handmixing in a mortar 4 g of the dispersion according to 2.4 with 6 g of the cream according to 3.1.
**5.4.** A cream containing hydrocortison-17-butyrate encapsulated in vesicles was produced by handmixing in a mortar 4 g of the dispersion according to 2.5 with 6 g of the cream according to 3.1.
**5.5.** A cream containing hydrocortison-17-butyrate without vesicles was prepared by handmixing in a mortar 10 mg of hydrocortison-17-butyrate with 10 g of the cream according to 3.1.

### Example 6

### Production of gels containing a dispersion of vesicles and disodium-fluorescein (free or encapsulated within the vesicles) or disodium-fluorescein alone

**6.1.** A gel containing encapsulated disodium-fluorescein in vesicles was produced by handmixing in a mortar 0.8 g of the dispersion according to 2.1 with 3.2 g of the gel according to 4.1. After 6 months the vesicles were still homogeneously distributed over the gel.
**6.2.** A gel containing free disodium-fluorescein and vesicles was produced by dissolving 1 mg disodium-fluorescein in 10 g of the gel according to 4.2. After 6 months the vesicles were still homogeneously distributed over the gel.
**6.3.** A gel containing disodium-fluorescein alone was produced by dissolving 1 mg of disodium-fluorescein in 10 g of the gel according to 4.1.

### Example 7

### In vitro budesonide release from creams containing a dispersion of vesicles

The creams according to 5.1 and 5.2 were compared in the following in vitro diffusion test.

The in vitro budesonide release experiments were performed at a temperature of 35°C using a perspex donor compartment in which the cream layer (1.8 mm) was separated from the aqueous acceptor phase (0.5% w/w CETOMACROGOL 1000®) by a cellulose-triacetate membrane (SM 14539, SARTORIUS®, The Netherlands) with a surface area of 52.8 cm². 100 ml acceptor phase was circulated through the acceptor chamber at a rate of about 1.8 ml/min. Samples of 1 ml were taken with 30 minutes intervals. After sampling the acceptor phase was filled up to 100 ml with 1 ml fresh acceptor phase. The budesonide concentrations in the samples were determined using HPLC. 50 µl samples were injected to the system with the aid of an automatic injector (GILSON® MODEL 231). The reversed phase HPLC column (CHROMSPHER® C18 100 x 3 mm, CHROMPACK®, The Netherlands) was eluted at 22°C with a mobile phase consisting of acetonitrile/water (35/65 v/v) at a flow rate of 0.8 ml/min. The column effluent was monitored at 242 nm (APPLIED BIOSYSTEMS® 757). The retention time of the budesonide was about 7 minutes.
Both creams were tested in duplicate, the maximal deviation between the results of one preparation being 8%.

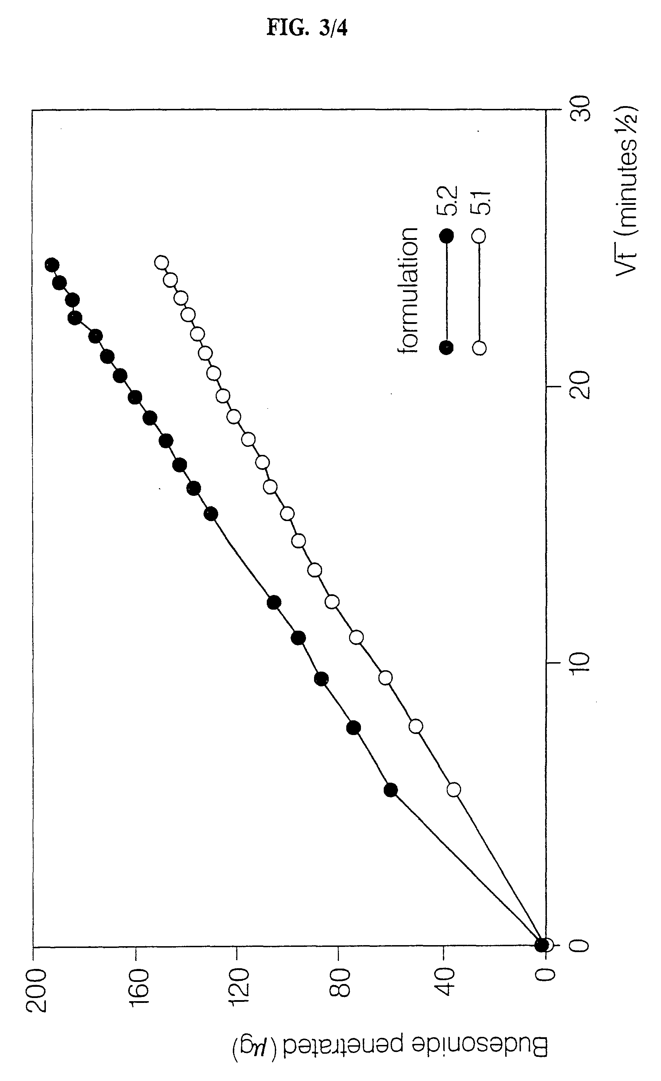

In Figure 3 the amount of budesonide penetrated through the membrane (as described above) is shown as a function of the squareroot of application time.
From these results it appeared that encapsulation of budesonide in vesicles within a cream significantly retarded the release of budesonide from such a cream.

### Example 8

### The influence of encapsulation on the fluorescence behaviour of disodium-fluorescein

The fluorescence behaviour of disodium-fluorescein in the gels according to 6.1, 6.2 and 6.3 were studied with reflected light fluorescence microscopy (RLFM).

A RLFM microscope (OLYMPUS® BH2, Japan) with blue light excitation was used to study the fluorescence pattern of gels 6.1, 6.2 and 6.3. The images of the microscope were registrated by a video camera (SONY® DXC-3000P) and taped with a video-recorder (SONY® VO 5800PS).
With the aid of the described apparatus it was possible to measure the time after which fluorescence disappeared within the gels. For the gels 6.1, 6.2, 6.3 this time was resp.: 469, 53 seconds and less then 1 second.
From these results it was clear that the highly water-soluble disodium-fluorescein was still encapsulated in the vesicles after dispersing them in a gel.

### Example 9

### In vivo corticosteroid release on human skin

The in vivo skin blanching effect of the corticosteroid creams according to 5.3, 5.4 and 5.5 were compared in a McKenzie-Stoughton test.

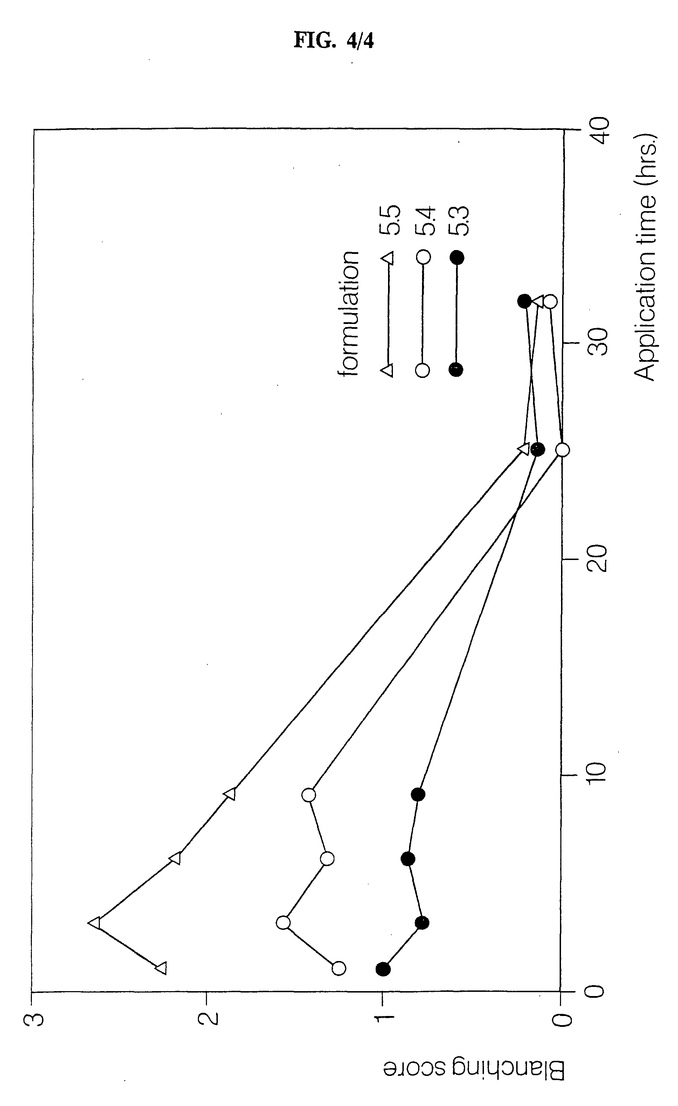

The test was conducted on a panel of non-patient volunteers (1 female and 7 males). Sites were marked on the flexor aspect of both forearms by light indentation with a circular punch of 15 mm diameter. Sites were at least 4 cm distant from wrist and elbow. The precoded preparations were applied to these sites according to a Latin square experimental design. Preparations were applied in amounts of 10 µl per site from a disposable pipette (Capilettor®). The application sites were then covered, but not occluded with a "guard", which was held in place with a ring of surgical tape (diameter: external 50/60 mm, internal 25 mm).
Dressings were removed and arms were washed with soap and lukewarm water 9 hours after application of the formulations. Accordingly, blanching at the various sites was visually assessed on an integral scale of 0-4 by two experienced scorers, working independently of each other. This was done at 1, 3, 6, 9, 25, and 32 hours after removal of the preparations. The experiment was conducted in a double-blind fashion.
The results of this test are shown in Figure 4 where the mean blanching scores are given as a function of time.
From these results it was concluded that the absolute amount of the steroid which reaches the deep skin department (where blanching is caused) from creams 5.1 and 5.2 were lower than those from the conventional cream 5.3. This was caused by the slower release and the retention of hydrocortison-17-butyrate due to its encapsulation.
The conventional cream had a peak score after 3 hours (2.64) the score rapidly decreased to 1.87 after 9 hours. Both creams with hydrocortison-17-butyrate encapsulated in multilamellar vesicles did not show such a fast decrease.
From these results it appeared that vesicles could be used to establish a sustained release of a medicament from a dermatological formulation and a retention in the upper skin regions.

## Claims (Claims for the following Contracting State(s): AT, BE, CH, LI, DE, DK, FR, GB, IT, LU, MC, NL, PT, SE)

1. A dispersion of vesicles, which encapsulate a non-polar phase, in a non-polar phase, the vesicles comprising a surfactant or mixture of surfactants, **characterised in that** the surfactant or mixture of surfactants is selected from the group consisting of sucrose fatty acid esters.

2. Dispersion of vesicles according to claim 1, **characterised in that** a lipophilic stabilising factor is present.

3. Dispersion of vesicles according to claim 2, wherein the lipophilic stabilising factor is a compound, selected from the group of sterols, such as (partially) hydrogenated 10,13-dimethyl cyclopentaphenanthrenes, optionally substituted by hydroxyl and carboxylic acid and esters thereof, such as cholesterol.

4. Dispersion of vesicles according to claim 2, wherein the lipophilic stabilising factor is a compound, selected from the group of retinoids, such as retinoic acid.

5. Dispersion of vesicles according to claim 2, wherein the lipophilic stabilising factor is a compound selected from the group of branched fatty alcohols, such as 2-hexyl octyl ethanol.

6. Dispersion of vesicles according to claim 2, wherein the lipophilic stabilising factor is a compound selected from the group of straight or branched chain (number of C-atoms: 4 or above) saturated aliphatic alcohol esters of aliphatic or aromatic dicarboxylic acids (number of C-atoms: 4 or above), such as dioctyl phthalate.

7. Dispersion of vesicles according to claim 2, wherein the lipophilic stabilising factor is a compound selected from the group of saturated and unsaturated fatty acids, such as palmitic acid.

8. Dispersion of vesicles according to claim 2, wherein the lipophilic stabilising factor is a compound selected from the group of straight and branched fatty acid esters with polyhydroxylic compounds, such as propylene glycol dipelargonate and diglycerol diisostearo stearate.

9. Dispersion of vesicles according to any one of claims 1-8, **characterised in that** it further comprises a hydrophilic stabilising factor, which is a polar liquid selected from the group consisting of water and compounds, having a molecular weight of up to 150 and having one or more functional groups able to form hydrogen-bridges, such as a hydroxyl, a mono- or di-substituted amino and a carboxylic acid group, such as ethanol and ethanolamine.

10. Dispersion of vesicles according to any one of claims 1-9, **characterised in that** the non-polar phase consists of a liquid paraffin or a volatile silicone oil.

11. Dispersion of vesicles according to any one of claims 1-10, **characterised in that** one or more hydrophilic (biologically) active agents are present in the non-polar phase and/or entrapped in the vesicles.

12. Dispersion of vesicles according to any one of claims 1-11, **characterised in that** one or more lipophilic (biologically) active agents are present in the non-polar phase and/or entrapped in the vesicles.

## Claims (Claims for the following Contracting State(s): ES and GR)

1. Process for the preparation of a dispersion of vesicles, which encapsulate a non-polar phase, in a non-polar phase, the vesicles comprising a surfactant or mixture of surfactants, **characterised in that** the surfactant or mixture of surfactants is selected from the group consisting of sucrose fatty acid esters.

2. Process according to claim 1, **characterised in that** a lipophilic stabilising factor As added.

3. Process according to claim 2, wherein the lipophilic stabilising factor is selected from the group of sterols, such as (partially) hydrogenated 10,13-dimethyl cyclopentaphenanthrenes, optionally substituted by hydroxyl and carboxylic acid and esters thereof, such as cholesterol.

4. Process according to claim 2, wherein the lipophilic stabilising factor is selected from the group of retinoids, such as retinoic acid.

5. Process according to claim 2, wherein the lipophilic stabilising factor is selected from the group of branched fatty alcohols, such as 2-hexyl octyl ethanol.

6. Process according to claim 2, wherein the lipophilic stabilising factor is selected from the group of straight or branched chain, (number of C-atoms: 4 or above) saturated aliphatic alcohol esters of aliphatic or aromatic dicarboxylic acids (number of C-atoms: 4 or above), such as dioctyl phthalate.

7. Process according to claim 2, wherein the lipophilic stabilising factor is selected from the group of saturated and unsaturated fatty acids, such as palmitic acid.

8. Process according to claim 2, wherein the lipophilic stabilising factor is selected from the group of straight and branched fatty acid esters with polyhydroxylic compounds, such as propylene glycol dipelargonate and diglycerol diisostearo stearate.

9. Process according to any one of claims 1-8, **characterised in that** a hydrophilic stabilising factor, which is a polar liquid selected from the group consisting of water and compounds, having a molecular weight of up to 150 and having one or more functional groups able to form hydrogen-bridges, such as a hydroxyl, a mono- or di-substituted amino and a carboxylic acid group, such as ethanol and ethanolamine, is added.

10. Process according to any one of claims 1-9, **characterised in that** as the non-polar phase a liquid paraffin or a volatile silicone oil is used.

11. Process according to any one of claims 1-10, **characterised in that** one or more hydrophilic (biologically) active agents are added to the non-polar phase and/or entrapped in the vesicles.

12. Process according to any one of claims 1-11, **characterised in that** one or more lipophilic (biologically) active agents are added to the non-polar phase and/or entrapped in the vesicles.

## Patentansprüche (Patentansprüche für folgende(n) Vertragsstaat(en): AT, BE, CH, LI, DE, DK, FR, GB, IT, LU, MC, NL, PT, SE)

1. Dispersion von Vesikeln, die eine nicht-polare Phase einkapseln, in einer nicht-polaren Phase, wobei die Vesikel ein grenzflächenaktives Mittel oder ein Gemisch aus grenzflächenaktiven Mitteln umfassen, **dadurch gekennzeichnet, dass** das grenzflächenaktive Mittel oder das Gemisch aus grenzflächenaktiven Mitteln ausgewählt ist aus der Gruppe, bestehend aus Saccharosefettsäureestern.

2. Dispersion von Vesikeln nach Anspruch 1, **dadurch gekennzeichnet, dass** ein lipophiler Stabilisierungsfaktor vorhanden ist.

3. Dispersion von Vesikeln nach Anspruch 2, wobei der lipophile Stabilisierungsfaktor eine Verbindung ist, ausgewählt aus der Gruppe der Sterole, wie (teilweise) hydrierte 10,13-Dimethylcyclopentaphenanthrene, gegebenenfalls substituiert durch Hydroxyl und Carbonsäure und Estern derselben, wie Cholesterin.

4. Dispersion von Vesikeln nach Anspruch 2, wobei der lipophile Stabilisierungsfaktor eine Verbindung ist, ausgewählt aus der Gruppe, bestehend aus Retinoiden, wie Retinsäure.

5. Dispersion von Vesikeln nach Anspruch 2, wobei der lipophile Stabilisierungsfaktor eine Verbindung ist, ausgewählt aus der Gruppe der verzweigten Fettalkohole, wie 2-Hexyloctylethanol.

6. Dispersion von Vesikeln nach Anspruch 2, wobei der lipophile Stabilisierungsfaktor eine Verbindung ist, ausgewählt aus der Gruppe geradkettiger oder verzweigtkettiger (Zahl der C-Atome: 4 oder darüber) gesättigter aliphatischer Alkoholester von aliphatischen oder aromatischen Dicarbonsäuren (Zahl der C-Atome: 4 oder darüber), wie z.B. Dioctylphthalat).

7. Dispersion von Vesikeln nach Anspruch 2, wobei der lipophile Stabilisierungsfaktor eine Verbindung ist, ausgewählt aus der Gruppe von gesättigten und ungesättigten Fettsäuren, wie Palmitinsäure.

8. Dispersion von Vesikeln nach Anspruch 2, wobei der lipophile Stabilisierungsfaktor eine Verbindung ist, ausgewählt aus der Gruppe geradkettiger oder verzweigtkettiger Fettsäureester mit Polyhydroxylverbindungen, wie Propylenglykoldipelargonat und Diglycerindiisostearostearat.

9. Dispersion von Vesikeln nach einem der Ansprüche 1 bis 8, **dadurch gekennzeichnet, dass** sie weiter einen hydrophilen Stabilisierungsfaktor enthält, der eine polare Flüssigkeit ist, ausgewählt aus der Gruppe, bestehend aus Wasser und Verbindungen mit einem Molekulargewicht bis zu 150 und mit einer oder mehreren funktionellen Gruppen, die Wasserstoffbrücken bilden können, wie eine Hydroxyl-, eine mono- oder disubstituierte Aminound eine Carbonsäuregruppe, wie Ethanol und Ethanolamin.

10. Dispersion von Vesikeln nach einem der Ansprüche 1 bis 9, **dadurch gekennzeichnet, dass** die nicht-polare Phase aus einem flüssigen Paraffin oder einem flüchtigen Silikonöl zusammengesetzt ist.

11. Dispersion von Vesikeln nach einem der Ansprüche 1 bis 10, **dadurch gekennzeichnet, dass** ein oder mehrere hydrophile (biologisch) aktive Mittel in der nicht-polaren Phase und/oder eingeschlossen in den Vesikeln vorhanden sind.

12. Dispersion von Vesikeln nach einem der Ansprüche 1 bis 11, **dadurch gekennzeichnet, dass** ein oder mehrere lipophile (biologisch) aktive Mittel in der nicht-polaren Phase und/oder eingeschlossen in den Vesikeln vorhanden sind.

## Patentansprüche (Patentansprüche für folgende(n) Vertragsstaat(en): ES, GR)

1. Verfahren zur Herstellung einer Dispersion von Vesikeln, die eine nicht-polare Phase einkapseln, in einer nicht-polaren Phase, wobei die Vesikel ein grenzflächenaktives Mittel oder ein Gemisch aus grenzflächenaktiven Mitteln umfassen, **dadurch gekennzeichnet, dass** das grenzflächenaktive Mittel oder das Gemisch aus grenzflächenaktiven Mitteln ausgewählt ist aus der Gruppe, bestehend aus Saccharosefettsäureestern.

2. Verfahren nach Anspruch 1, **dadurch gekennzeichnet, dass** ein lipophiler Stabilisierungsfaktor hinzugefügt wird.

3. Verfahren nach Anspruch 2, wobei der lipophile Stabilisierungsfaktor ausgewählt ist aus der Gruppe der Sterole, wie (teilweise) hydrierte 10,13-Dimethylcyclopentaphenanthrene, gegebenenfalls substituiert durch Hydroxyl und Carbonsäure und Estern derselben, wie Cholesterin.

4. Verfahren nach Anspruch 2, wobei der lipophile Stabilisierungsfaktor ausgewählt ist aus der Gruppe, bestehend aus Retinoiden, wie Retinsäure.

5. Verfahren nach Anspruch 2, wobei der lipohile Stabilisierungsfaktor ausgewählt ist aus der Gruppe der verzweigten Fettalkohole, wie 2-Hexyloctylethanol.

6. Verfahren nach Anspruch 2, wobei der lipophile Stabilisierungsfaktor ausgewählt ist aus der Gruppe geradkettiger oder verzweigtkettiger (Zahl der C-Atome: 4 oder darüber) gesättigter aliphatischer Alkoholester von aliphatischen oder aromatischen Dicarbonsäuren (Zahl der C-Atome: 4 oder darüber), wie z.B. Dioctylphthalat).

7. Verfahren nach Anspruch 2, wobei der lipophile Stabilisierungsfaktor ausgewählt ist aus der Gruppe von gesättigten und ungesättigten Fettsäuren, wie Palmitinsäure.

8. Verfahren nach Anspruch 2, wobei der lipophile Stabilisierungsfaktor ausgewählt ist aus der Gruppe geradkettiger oder verzweigtkettiger Fettsäureeester mit Polyhydroxylverbindungen, wie Propylenglykoldipelargonat und Diglycerindiisostearostearat.

9. Verfahren nach einem der Ansprüche 1 bis 8, **dadurch gekennzeichnet, dass** ein hydrophiler Stabilisierungsfaktor hinzugefügt wird, der eine polare Flüssigkeit ist, ausgewählt aus der Gruppe, bestehend aus Wasser und Verbindungen mit einem Molekulargewicht bis zu 150 und mit einer oder mehreren funktionellen Gruppen, die Wasserstoffbrücken bilden können, wie eine Hydroxyl-, eine mono- oder disubstituierte Amino- und eine Carbonsäuregruppe, wie Ethanol und Ethanolamin.

10. Verfahren nach einem der Ansprüche 1 bis 9, **dadurch gekennzeichnet, dass** zur Herstellung der nicht-polaren Phase flüssiges Paraffin oder ein flüchtiges Silikonöl verwendet wird.

11. Verfahren nach einem der Ansprüche 1 bis 10, **dadurch gekennzeichnet, dass** ein oder mehrere hydrophile (biologisch) aktive Mittel in der nicht-polaren Phase und/oder eingeschlossen in den Vesikeln hinzugefügt werden.

12. Verfahren nach einem der Ansprüche 1 bis 11, **dadurch gekennzeichnet, dass** ein oder mehrere lipophile (biologisch) aktive Mittel in der nicht-polaren Phase und/oder eingeschlossen in den Vesikeln hinzugefügt werden.

## Revendications (Revendications pour l'(les) Etat(s) contractant(s) suivant(s): AT, BE, CH, LI, DE, DK, FR, GB, IT, LU, MC, NL, PT, SE)

1. Dispersion de vésicules, lesquelles encapsulent une phase non polaire, dans une phase non polaire, les vésicules étant composées d'un surfactant ou d'un mélange de surfactants, **caractérisée en ce que** le surfactant ou le mélange de surfactants est sélectionné dans le groupe composé d'esters d'acides gras du sucrose.

2. Dispersion de vésicules suivant la revendication 1, **caractérisée en ce qu'**un facteur stabilisateur lipophile est présent.

3. Dispersion de vésicules suivant la revendication 2, dans laquelle le facteur stabilisateur lipophile est un composé sélectionné dans le groupe de stérols, tels que les 10,13-diméthylcyclopentaphénanthrènes (partiellement) hydrogénés, éventuellement substitués par des hydroxyles et des acides carboxyliques et leurs esters, comme le cholestérol.

4. Dispersion de vésicules suivant la revendication 2, dans laquelle le facteur stabilisateur lipophile est un composé sélectionné dans le groupe des rétinoïdes, tels que l'acide rétinoique.

5. Dispersion de vésicules suivant la revendication 2, dans laquelle le facteur stabilisateur lipophile est un composé sélectionné dans le groupe des alcools gras ramifiés, comme le 2-hexyloctyléthanol.

6. Dispersion de vésicules suivant la revendication 2, dans laquelle le facteur stabilisateur lipophile est un composé sélectionné dans le groupe des esters d'alcools aliphatiques saturés à chaîne linéaire ou ramifiée (nombre d'atomes de carbone: 4 ou plus) d'acides dicarboxyliques aliphatiques ou aromatiques (nombre d'atomes de carbone: 4 ou plus), tels que le phtalate de dioctyle.

7. Dispersion de vésicules suivant la revendication 2, dans laquelle le facteur stabilisateur lipophile est un composé sélectionné dans le groupe des acides gras saturés et insaturés, tels que l'acide palmitique.

8. Dispersion de vésicules suivant la revendication 2, dans laquelle le facteur stabilisateur lipophile est un composé sélectionné dans le groupe des esters d'acides gras linéaires et ramifiés avec des composés polyhydroxylés, tels que le dipélargonate de propylèneglycol et le diisostéarostéarate de diglycérol.

9. Dispersion de vésicules suivant l'une quelconque des revendications 1 à 8, **caractérisée en ce qu'**elle comprend en outre un facteur stabilisateur hydrophile, qui est un liquide polaire sélectionné dans le groupe comprenant l'eau et des composés ayant un poids moléculaire de jusqu'à 150 et possédant un ou plusieurs groupements fonctionnels capables de former des ponts hydrogène, tels qu'un groupement hydroxyle, amino mon ou disubstitué et acide carboxylique, comme l'éthanol et l'éthanolamine.

10. Dispersion de vésicules suivant l'une quelconque des revendications 1 à 9, **caractérisée en ce que** la phase non polaire est composée d'une paraffine liquide ou d'une huile de silicone volatile.

11. Dispersion de vésicules suivant l'une quelconque des revendications 1 à 10, **caractérisée en ce que** un ou plusieurs agents hydrophiles biologiquement actifs sont présents dans la phase non polaire et/ou piégés dans les vésicules.

12. Dispersion de vésicules suivant l'une quelconque des revendications 1 à 11, **caractérisée en ce que** un ou plusieurs agents lipophiles biologiquement actifs sont présents dans la phase non polaire et/ou piégés dans les vésicules.

## Revendications (Revendications pour l'(les) Etat(s) contractant(s) suivant(s): ES, GR)

1. Procédé de préparation d'une dispersion de vésicules, lesquelles encapsulent une phase non polaire, dans une phase non polaire, les vésicules étant composées d'un surfactant ou d'un mélange de surfactants, **caractérisé en ce que** le surfactant ou le mélange de surfactants est sélectionné dans le groupe composé d'esters d'acides gras du sucrose.

2. Procédé suivant la revendication 1, **caractérisé en ce que** l'on ajoute un facteur stabilisateur lipophile.

3. Procédé suivant la revendication 2, dans lequel le facteur stabilisateur lipophile est sélectionné dans le groupe de stérols, tels que les 10,13-diméthylcyclopentaphénanthrènes (partiellement) hydrogénés, éventuellement substitués par des hydroxyle et des acides carboxyliques et leurs esters, comme le cholestérol.

4. Procédé suivant la revendication 2, dans lequel le facteur stabilisateur lipophile est sélectionné dans le groupe des rétinoides, tels que l'acide rétinoique.

5. Procédé suivant la revendication 2, dans lequel le facteur stabilisateur lipophile est sélectionné dans le groupe des alcools gras ramifiés, comme le 2-hexyloctyléthanol.

6. Procédé suivant la revendication 2, dans lequel le facteur stabilisateur lipophile est sélectionné dans le groupe des esters d'alcools aliphatiques saturés à chaîne linéaire ou ramifiée (nombre d'atomes de carbone: 4 ou plus) d'acides dicarboxyliques aliphatiques ou aromatiques (nombre d'atomes de carbone: 4 ou plus), tels que le phtalate de dioctyle.

7. Procédé suivant la revendication 2, dans lequel le facteur stabilisateur lipophile est sélectionné dans le groupe des acides gras saturés et insaturés, tels que l'acide palmitique.

8. Procédé suivant la revendication 2, dans lequel le facteur stabilisateur lipophile est sélectionné dans le groupe des esters d'acides gras linéaires et ramifiés avec des composés polyhydroxylés, tels que le dipélargonate de propylèneglycol et le diisostéarostéarate de diglycérol.

9. Procédé suivant l'une quelconque des revendications 1 à 8, **caractérisé en ce qu'**on ajoute un facteur stabilisateur hydrophile, qui est un liquide polaire sélectionné dans le groupe comprenant l'eau et des composés ayant un poids moléculaire de jusqu'à 150 et possédant un ou plusieurs groupements fonctionnels capables de former des ponts hydrogène, tels qu'un groupement hydroxyle, amino mono ou disubstitué et acide carboxylique, comme l'éthanol et l'éthanolamine.

10. Procédé suivant l'une quelconque des revendications 1 à 9, **caractérisé en ce que** l'on utilise une paraffine liquide ou une huile de silicone volatile en tant que phase non polaire.

11. Procédé suivant l'une quelconque des revendications 1 à 10, **caractérisé en ce que** un ou plusieurs agents hydrophiles (biologiquement) actifs sont ajoutés à la phase non polaire et/ou piégés dans les vésicules.

12. Procédé suivant l'une quelconque des revendications 1 à 11, **caractérisé en ce que** un ou plusieurs agents lipophiles (biologiquement) actifs sont ajoutés à la phase non polaire et/ou piégés dans les vésicules.
